# EUROPÄISCHE PATENTANMELDUNG

(11) **EP 4 725 510 A1**
(43) Veröffentlichungstag der Anmeldung: **15.04.2026**
(21) Anmeldenummer: 25208085.8
(22) Anmeldetag: 10.10.2025
(51) Int. Cl.: A61L 9/12

(54) **RAUMBEDUFTUNGSVORRICHTUNG, UND VERFAHREN ZUR RAUMBEDUFTUNG VON RÄUMEN IN GEBÄUDEN**

(30) Priorität: 11.10.2024 DE 102024129529
(71) Anmelder: Van Bömmel, Christoph, 41063 Mönchengladbach (DE)
(72) Erfinder: Van Bömmel, Christoph, 41063 Mönchengladbach (DE)
(74) Vertreter: Dammertz, Ulrich

(57) **Zusammenfassung**

Die Erfindung betrifft eine Raumbeduftungsvorrichtung (10) und ein Verfahren zur Raumbeduftung von Räumen in Gebäuden. Die Raumbeduftungsvorrichtung (10) umfasst einen Verdunstungskörper (12) mit einer Vielzahl von Fasern (F), wobei der Verdunstungskörper (12) eine Kapillarwirkung aufweist und zur Aufnahme von Duft- oder Geruchsstoffen in flüssiger Form geeignet ist. Der Verdunstungskörper (12) ist aus einem Textil-Material gebildet ist oder weist zumindest ein Textil-Material auf.

## Beschreibung

Die Erfindung betrifft eine Raumbeduftungsvorrichtung nach dem Oberbegriff von Anspruch 1, und ein Verfahren zur Raumbeduftung von Räumen in Gebäuden.

Nach dem Stand der Technik sind zwecks einer Raumbeduftung sogenannte Duftstäbe aus Rattan- oder Weidenstäbchen bekannt, wie es beispielweise in den Druckschriften WO 2016/150854 A1 und WO 2018/072043 A1 diskutiert wird. Bei diesen Duftstäben wird eine mit Duftstoffen versehene Flüssigkeit durch den Kapillareffekt aufgesogen und über die Poren der Stäbchen an die Umgebung abgegeben, um dadurch den Raumduft zu beeinflussen. Hierbei sind die Duftstäbe mit einem Ende davon in einen Behälter eingebracht, welcher mit der mit Duftstoffen versehenen Flüssigkeit gefüllt ist. Das entgegensetzte Ende der Duftstäbe ragt aus dem Behälter heraus, wo dann der Duftstoff per Verdunstung der Flüssigkeit an die Raumluft abgegeben wird.

Natürliche Duftstäbe, die beispielsweise aus Rattan oder Balsaholz bestehen, haben den Nachteil, dass deren Dimensionierung von der Verfügbarkeit des natürlichen Ausgangsmaterials abhängig ist. Beispielsweise kann eine vom Anwender gewünschte Dimensionierung, die über ein übliches Tischformat hinausgeht, mit empfindlich hohen Kosten verbunden sein, oder ist ggf. gar nicht realisierbar. Des Weiteren besteht ein Nachteil von herkömmlich bekannten natürlichen Duftstäbe darin, dass diese in der Regel nur für einen konkreten Duft eingesetzt werden, wobei ein Wechsel zu einem anderen Duft mit dem gleichen Duftstäbchen nicht vorgesehen ist bzw. nicht zufriedenstellend funktioniert. Als ebenfalls nachteilig ist anzusehen, dass handelsübliche Duftstäbe, zumindest nach einer gewissen Einsatzdauer, Wegwerfartikel sind.

Entsprechend liegt der Erfindung die Aufgabe zugrunde, eine Raumbeduftung von Räumen in Gebäuden mit einfachen Mitteln zu optimieren.

Die obige Aufgabe wird durch eine Raumbeduftungsvorrichtung mit den Merkmalen von Anspruch 1 als auch durch ein Verfahren mit den Merkmalen von Anspruch 11 gelöst. Vorteilhafte Weiterbildungen der Erfindung sind in den abhängigen Ansprüchen definiert.

Eine Raumbeduftungsvorrichtung nach der vorliegenden Erfindung dient zur Abgabe eines Duftstoffs an die Umgebung insbesondere in Form von geschlossenen Räumen in Gebäuden. Die erfindungsgemäße Raumbeduftungsvorrichtung umfasst einen Verdunstungskörper mit einer Vielzahl von Fasern, wobei der Verdunstungskörper eine Kapillarwirkung aufweist und zur Aufnahme von Duftstoffen in flüssiger Form geeignet ist. Die Erfindung ist durch das Merkmal gekennzeichnet, dass der Verdunstungskörper aus einem Textil-Material gebildet ist oder zumindest ein Textil-Material aufweist.

Gemäß einer vorteilhaften Weiterbildung der Erfindung ist vorgesehen, dass die Raumbeduftungsvorrichtung einen Behälter für eine zumindest einen Duftstoff enthaltende Flüssigkeit aufweist. Hierbei ist der Verdunstungskörper in Form des Textil-Materials mit seinem unteren Ende in eine Öffnung des Behälters einbringbar und ragt mit seinem oberen Ende aus der Öffnung des Behälters heraus.

Bei einem bestimmungsgemäßen Einsatz der erfindungsgemäßen Raumbeduftungsvorrichtung ist vorgesehen, dass der Verdunstungskörper mit einem Ende davon in den Behälter eingebracht wird, der mit einer aromatisierten Flüssigkeit oder dergleichen gefüllt ist. In Folge der Kapillarwirkung steigt die Flüssigkeit an bzw. in dem Verdunstungskörper nach oben in Richtung seines entgegengesetzten Endes, der aus dem Behälter nach oben herausragt. Hierbei wird dann der Duft- bzw. Geruchsstoff, der in der aromatisierten Flüssigkeit enthalten ist, bei einer Verdunstung der Flüssigkeit an dem oberen exponierten Teil des Verdunstungskörpers, der aus dem Behälter herausragt, an die Umgebung freigesetzt und insoweit eine gewünschte Raumbeduftung realisiert.

Der Erfindung liegt die wesentliche Erkenntnis zugrunde, dass der Verdunstungskörper eine doppelte bzw. integrierte Funktion erfüllt: Einerseits wird mit diesem Verdunstungskörper dank der Kapillarwirkung, die wegen der Vielzahl der zugehörigen Fasern vorliegt, der Effekt erreicht, dass Flüssigkeit von einem unteren Ende des Verdunstungskörpers entlang seiner Längs-erstreckung in Richtung seines oberen Endes transportiert wird, und zwar selbsttätig, ohne den Einsatz von zusätzlicher Energie und/oder von technischen Hilfsmitteln in Form von Pumpen oder dergleichen. Des Weiteren dient der besagte Verdunstungskörper, dem Wortsinn seiner Bezeichnung entsprechend, auch zu dem Zweck, dass die Flüssigkeit aus bzw. von diesem Verdunstungskörper nach außen hin verdunsten kann und dabei dann die Duft- bzw. Geruchsstoffe, die in der Flüssigkeit enthalten sind, bestimmungsgemäß an die Umgebung eines Raums abgegeben werden.

Im Sinne der vorliegenden Erfindung wird mit dem Merkmal "Textil-Material" ein jegliches Material mit einer Vielzahl von Fasern verstanden, das aus miteinander verbundenen oder verflochtenen Fasern besteht. Jeder gewebte oder gestrickte Stoff stellt erfindungsgemäß ein Textil dar. Erfindungsgemäß können die Fasern, aus denen der Verdunstungskörper hergestellt ist, aus Naturfasern, Kunstfasern, Glasfasern, Garn, Faden, Naturseide, Viskose, Chemiefasern, elastischen Fasern und/oder aus Metall- bzw. Stahlfäden bestehen.

Gemäß einer vorteilhaften Weiterbildung der vorliegenden Erfindung weist der Verdunstungskörper eine Längserstreckung auf, wobei die Kapillarwirkung des Verdunstungskörpers zumindest entlang seiner Längserstreckung vorliegt. Zweckmäßigerweise kann für den Verdunstungskörper die genannte Kapillarwirkung auch omnidirektional vorliegen, d.h. nicht nur entlang einer Längserstreckung, sondern auch in einer Richtung quer bzw. orthogonal zur Längserstreckung. Für diesen Fall ist erfindungsgemäß und vorteilhaft gewährleistet, dass sich eine Flüssigkeit in verschiedenen Richtungen innerhalb des Verdunstungskörpers verteilt, falls beispielsweise ein Ende des Verdunstungskörpers in Kontakt mit der Flüssigkeit gelangt bzw. darin eingetaucht wird. Falls der Verdunstungskörper in Form eines Strumpfs ausgebildet ist, wird durch die besagte omnidirektionale Kapillarwirkung sichergestellt, dass die Flüssigkeit innerhalb des Verdunstungskörpers nicht nur von seinem unteren Ende in Richtung des oberen Endes, sondern auch entlang des Umfangs des Verdunstungskörpers gleichmäßig transportiert bzw. verteilt wird.

In vorteilhafter Weiterbildung der Erfindung kann die Raumbeduftungsvorrichtung ein längliches stabförmiges Element umfassen, wobei der Verdunstungskörper in Form des Textil-Materials an einer Außenumfangsfläche des stabförmigen Elements angebracht ist, so dass der Verdunstungskörper gegenüber der Umgebung exponiert ist. Zweckmäßigerweise kann hierbei der Verdunstungskörper in Form des Textil-Materials die Außenumfangsfläche des stabförmigen Elements entlang seiner Längserstreckung vollständig bedecken. Hierbei kann vorgesehen sein, dass das Textil-Material in Form eines Strumpfs ausgebildet ist, wobei dieser Strumpf zur Anbringung an dem stützenden stabförmigen Element hierüber gezogen wird. Anders ausgedrückt, befindet sich bei dieser Ausführungsform dann das strumpfförmige Textil-Material an der Außenumfangsfläche eines stabförmigen Elements und wird hiervon geeignet in seiner Längsrichtung gestützt.

Das vorstehend genannte stabförmige Element verleiht dem Verdunstungskörper in Form des Textil-Materials eine ausreichende Stabilität in Richtung seiner Längserstreckung, nämlich dadurch, dass dieses Textil-Material an der Außenumfangsfläche des stabförmigen Elements angebracht ist. Hierbei kann vorgesehen sein, dass der Verdunstungskörper in Form des Textil-Materials das stabförmige Element nach Art eines Strumpfs umschließt. Anders ausgedrückt, umschließt der Verdunstungskörper in Form des Textil-Materials das stabförmige Element in einer Ebene senkrecht zu dessen Längsachse vollständig.

Das stabförmige Element kann aus beliebigem Material bestehen bzw. hergestellt sein, beispielweise aus Holz, Glas, Stein, Metall, Kunststoff, Keramik, Gips etc. Für die Beschaffenheit des stabförmigen Elements ist für die vorliegende Erfindung einzig von Bedeutung, dass das Material, aus dem dieses stabförmige Element besteht bzw. hergestellt ist, eine ausreichende Eigenstabilität bzw. Knick- und somit Standfestigkeit aufweist, so dass der Verdunstungskörper in Form des Textil-Materials an einer Außenumfangsfläche des stabförmigen Elements angebracht werden kann, beispielsweise nach Art eines Strumpfs.

Gemäß einer vorteilhaften Weiterbildung der Erfindung kann das stabförmige Element zumindest in einem Segment entlang seiner Längsachse ein im Querschnitt hohl ausgebildetes Rohr sein. Zweckmäßigerweise besteht das stabförmige Element entlang seiner gesamten Längsachse aus einem im Querschnitt hohl ausgebildeten Rohr. Eine solche rohrförmige Beschaffenheit des stabförmigen Elements führt neben günstigen Herstellungskosten auch zu dem Vorteil, dass das Eigengewicht des stabförmigen Elements bei größeren Abmessungen, z.B. einer Länge von mehr als 50 cm, relativ gering bleibt und sich damit dann das stabförmige Element weiterhin einfach handhaben lässt.

Die vorstehend genannte mögliche rohrförmige Beschaffenheit des stabförmigen Elements führt weiterhin zu dem praktischen Vorteil, dass sich der Verdunstungskörper in Form des Textil-Materials an den Stirnseiten des Rohrs, wenn dort das Textil-Material übersteht, dann in einfacher Weise in den Innenraum des Rohrs hineindrücken lässt und mittels eines Stopfens an bzw. in der Stirnseite des Rohrs befestigt werden kann.

Die Möglichkeit, dass bei Vorliegen eines stabförmigen Elements der Verdunstungskörper in Form des Textil-Materials wie erläutert an der Außenumfangsfläche stabförmigen Elements angebracht werden kann, sieht im Umkehrschluss auch die Möglichkeit vor, dass nach Gebrauch der erfindungsgemäßen Raumbeduftungsvorrichtung dieses Textil-Material von dem stabförmigen Element wieder getrennt bzw. entfernt werden kann, beispielsweise zwecks einer Reinigung und/oder Reparatur, falls erforderlich. In dieser Weise ist eine mehrfache Verwendung des Verdunstungskörpers in Form des Textil-Materials gewährleistet, auch im Sinne einer ökologischen Nachhaltigkeit.

Gemäß einer alternativen Ausführungsform der erfindungsgemäßen Raumbeduftungsvorrichtung kann vorgesehen sein, dass die Fasern des Verdunstungskörpers in Form des Textil-Materials derart beschaffen sind, dass der Verdunstungskörper, falls er mit seiner Längserstreckung vertikal aufgestellt ist, in Richtung seiner Längserstreckung eine ausreichende Knickfestigkeit aufweist und dadurch selbstständig standfest ist. Für diesen Fall kann die Verwendung eines separaten stabförmigen Elements zwecks einer Stützung des Verdunstungskörpers in Richtung seiner Längserstreckung entfallen.

Im Hinblick darauf, dass die Fasern des Verdunstungskörpers der erfindungsgemäßen Raumbeduftungsvorrichtung nicht natürlichen Ursprungs sind, sondern Teil des charakteristischen Textil-Materials sind, aus denen der Verdunstungskörper gebildet ist oder aus denen der Verdunstungskörper zumindest teilweise bestehen kann, ist es mittels der vorliegenden Erfindung auch möglich, den Verdunstungskörper in einer vergleichsweise großen Dimensionierung herzustellen, beispielsweise mit einer Längserstreckung von mehr als 100 cm, oder ggf. noch größer. Falls hierbei zur Stützung des Verdunstungskörpers ein stabförmiges Element zum Einsatz kommt, versteht sich, dass dieses an die Längserstreckung des Verdunstungskörpers angepasst ist, beispielsweise in der genannten Länge von 100 cm (oder ggf. noch größer). Eine solche vergleichsweise große Dimensionierung des Verdunstungskörpers (und ggf. auch eines hierbei eingesetzten stabförmigen Elements, an dessen Außenumfangsfläche der Verdunstungskörper in Form des Textil-Materials angebracht ist) mit einer Längserstreckung von beispielsweise ≥ 100 cm ist für den Fall zweckmäßig, wenn mittels der vorliegenden Erfindung große Räume beduftet werden, beispielsweise eine Veranstaltungshalle, eine Hotellobby oder dergleichen. In diesem Zusammenhang versteht sich, dass dann auch ein Behälter, in den der Verdunstungskörper mit seinem unteren Ende davon eingebracht wird, entsprechend groß ist und an die Dimensionierung des Verdunstungskörpers angepasst ausgebildet ist

Des Weiteren sieht die vorliegende Erfindung auch ein Verfahren zur Raumbeduftung insbesondere von geschlossenen Räumen in Gebäuden vor. Bei diesem Verfahren wird ein Verdunstungskörper, der aus einem Textil-Material gebildet ist oder zumindest ein Textil-Material aufweist und dank einer Vielzahl von Fasern entlang seiner Längserstreckung eine Kapillarwirkung aufweist, mit seinem unteren Ende in eine Öffnung eines Behälters, der mit einer einen Duftstoff enthaltenden Flüssigkeit gefüllt ist, eingebracht, wobei der Verdunstungskörper mit seinem entgegengesetzten oberen Ende aus der Öffnung des Behälters herausragt. Hierbei ist der Verdunstungskörper in einem Längenabschnitt davon, der sich oberhalb der Öffnung des Behälters und somit außerhalb des Behälters befindet, gegenüber der Umgebung exponiert.

Bei dem soeben genannten Verfahren gemäß der vorliegenden Erfindung ist in Bezug auf den Verdunstungskörper zumindest in dessen Längenabschnitt, der sich oberhalb der Öffnung des Behälters befindet und gegenüber der Umgebung exponiert ist, eine Verdunstung der Flüssigkeit gewährleistet, so dass es in diesem Längenabschnitt des Verdunstungskörpers, neben einem Transport der Flüssigkeit in Richtung des oberen freien Endes des Verdunstungskörpers in Folge der Kapillarwirkung, auch zu einem Verdunsten der Flüssigkeit und somit zu einem Freisetzen der darin enthaltenen Duft- und Geruchsstoffen kommt.

In Bezug auf das vorstehend genannte erfindungsgemäße Verfahren versteht sich, dass dieses mit einer Raumbeduftungsvorrichtung gemäß einer der vorstehend genannten erfindungsgemäßen Ausführungsformen durchgeführt werden kann.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist nachfolgend anhand von bevorzugten Ausführungsformen in der Zeichnung schematisch dargestellt und wird unter Bezugnahme auf die Zeichnung ausführlich beschrieben. Es zeigen:
- Fig 1: eine vereinfachte Draufsicht auf eine erfindungsgemäße Raumbeduftungsvorrichtung und deren zugehörigen Verdunstungskörper,
- Fig. 2a: eine vereinfachte Seitenansicht eines stabförmigen Elements, der Teil der erfindungsgemäßen Raumbeduftungsvorrichtung sein kann,
- Fig. 2b: eine Seitenansicht einer erfindungsgemäßen Raumbeduftungsvorrichtung mit ihrem zugehörigen Verdunstungskörper gemäß einer weiteren Ausführungsform, die zur Verwendung mit dem stabförmigen Element von Fig. 2a vorgesehen ist,
- Fig. 2c: die erfindungsgemäße Raumbeduftungsvorrichtung, wenn der Verdunstungskörper gemäß Fig. 2b an einer Außenumfangsfläche des stabförmigen Elements von Fig. 2a angebracht ist,
- Fig. 3: eine Querschnittansicht der Raumbeduftungsvorrichtung entlang der Linie A-A von Fig. 2c,
- Fig. 4: eine Längsschnittansicht der Raumbeduftungsvorrichtung gemäß Fig. 2c,
- Fig. 5: eine vereinfachte Seitenansicht eines Behälters, der Teil der erfindungsgemäßen Raumbeduftungsvorrichtung ist,
- Fig. 6: eine Seitenansicht des Behälters von Fig. 5, wenn darin eine Raumbeduftungsvorrichtung gemäß Fig. 2c mit einem Ende davon eingebracht ist, und
- Fig. 7: eine perspektivisch vereinfachte Raumansicht mit darin gezeigten weiteren Ausführungsformen der erfindungsgemäßen Raumbeduftungsvorrichtung, wobei ein Verdunstungskörper gemäß Fig. 1 und ein Behälter gemäß Fig. 5 zum Einsatz kommen.

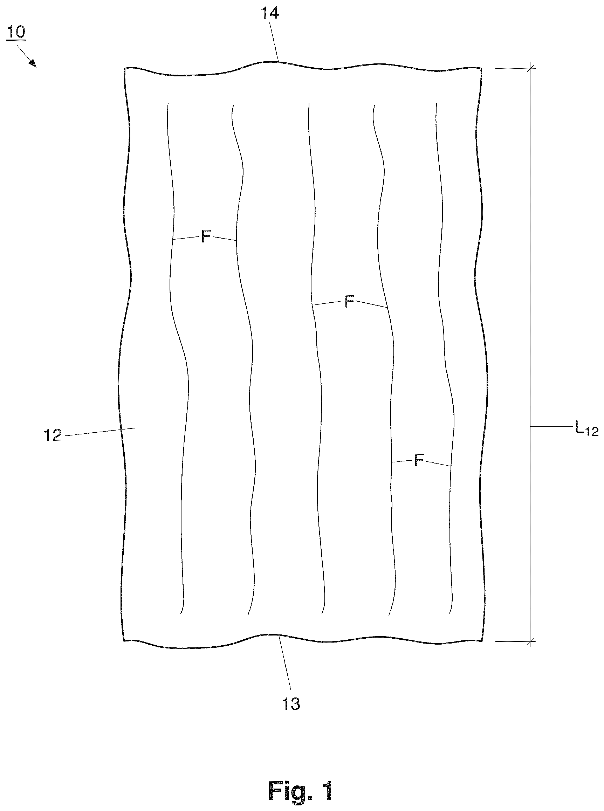

Nachstehend sind unter Bezugnahme auf die Fig. 1-7 bevorzugte Ausführungsformen einer erfindungsgemäßen Raumbeduftungsvorrichtung 10 und eines entsprechenden Verfahrens dargestellt und erläutert, mit der bzw. dem das Ausbringen bzw. Verteilen eines Dufts vorzugsweise in geschlossenen Räumen eines Gebäudes möglich ist. Gleiche Merkmale in der Zeichnung sind jeweils mit gleichen Bezugszeichen versehen. An dieser Stelle wird gesondert darauf hingewiesen, dass die Zeichnung lediglich vereinfacht und insbesondere ohne Maßstab dargestellt ist.

Fig. 1 zeigt eine Seitenansicht einer erfindungsgemäßen Raumbeduftungsvorrichtung 10. Bei dieser Ausführungsform ist ein Verdunstungskörper 12 der Raumbeduftungsvorrichtung 10 als längliches Textil ausgebildet, mit einem unteren Ende 13 und einem oberen Ende 14, und einer Längserstreckung L₁₂, die zwischen dem oberen und unteren Ende 13, 14 vorliegt.

Der Verdunstungskörper 12 ist aus einem Textil-Material mit einer Vielzahl von Fasern F ausgebildet. Diesbezüglich ist für die Funktionsweise der vorliegenden Erfindung von Bedeutung, dass der Verdunstungskörper 12 zumindest entlang seiner Längserstreckung L₁₂ eine Kapillarwirkung aufweist. In Folge dieser Kapillarwirkung ist gewährleistet, dass Flüssigkeit, falls das untere Ende 13 des Verdunstungskörpers 12 in Kontakt mit einer solchen Flüssigkeit kommt, dann stetig und ohne die Zufuhr einer externen zusätzlichen Energie nach oben in Richtung des oberen Endes 14 des Verdunstungskörpers 12 transportiert wird.

In Bezug auf den Verdunstungskörper 12 gemäß Fig. 1 wird ergänzend darauf hingewiesen, dass die besagte Kapillarwirkung auch orthogonal bzw. quer zur Längserstreckung L₁₂ des Verdunstungskörpers 12 vorliegen kann. Somit ist ein omnidirektionaler Transport von Flüssigkeit innerhalb des Verdunstungskörpers 12 gewährleistet, falls das untere Ende 13 in Kontakt mit Flüssigkeit gelangt. Dies wird nachfolgend noch gesondert erläutert.

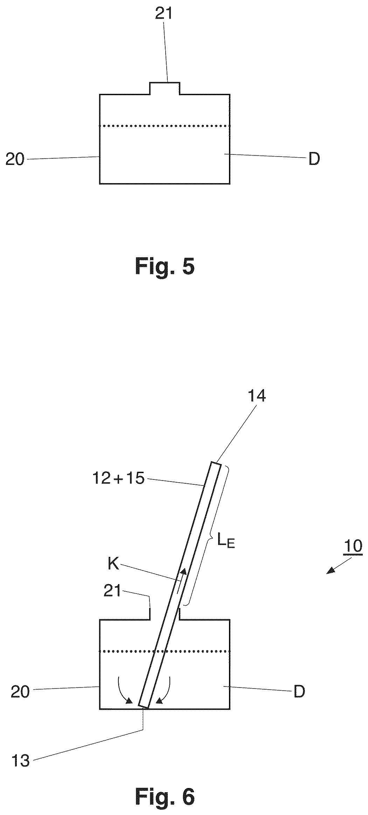

Beim praktischen Gebrauch der erfindungsgemäßen Raumbeduftungsvorrichtung 10 ist vorgesehen, dass hierbei ein Behälter 20 zum Einsatz kommt, der mit einer Flüssigkeit, welche zumindest einen Duft- oder Geruchsstoff enthält, gefüllt ist. Ein solcher Behälter 20 ist prinzipiell vereinfacht in Fig. 5 in einer Seitenansicht gezeigt. Die zumindest einen Duft- oder Geruchsstoff enthaltende Flüssigkeit ist in der Fig. 5 mit dem Bezugszeichen "D" bezeichnet, wobei der Füllstand dieser Flüssigkeit D in dem Behälter 20 mit einer horizontalen gestrichelten Linie symbolisiert ist.

Der Behälter 20 (vgl. Fig. 5) weist an seinem oberen Ende eine Öffnung 21 auf. Durch diese Öffnung 21 ist es möglich, die Raumbeduftungsvorrichtung 10 mit ihrem zugehörigen Verdunstungskörper 12 und dessen unteren Ende 13 in den Behälter 20 einzubringen. Nachstehend ist dies anhand von verschiedenen Ausführungsform der Erfindung erläutert.

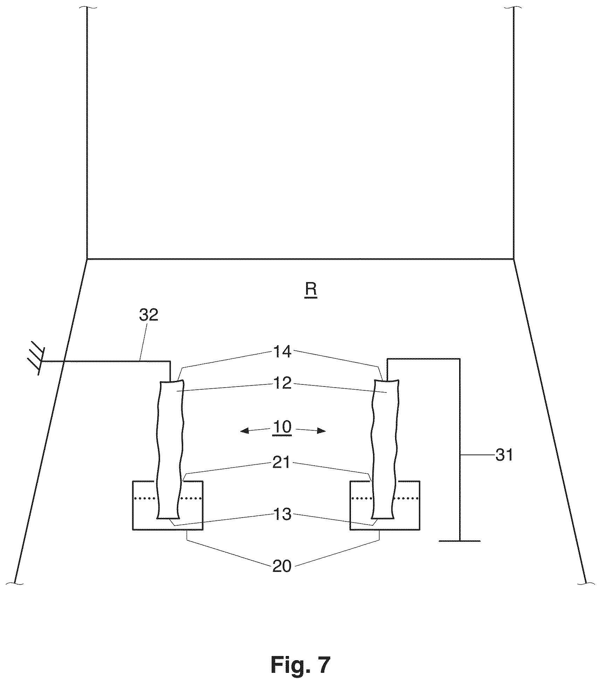

Fig. 7 veranschaulicht prinzipiell vereinfacht eine Perspektivansicht eines Raums R in einem Gebäude, mit zwei darin gezeigten Ausführungsformen der erfindungsgemäßen Raumbeduftungsvorrichtung 10. Diesen beiden Ausführungsformen ist gemeinsam, dass hierbei der Verdunstungskörper 12 der erfindungsgemäßen Raumbeduftungsvorrichtung 10 ohne weitere Stütz- oder Verstärkungselemente eingesetzt wird, sondern stattdessen nur mit einer geeigneten Halterung, an der jeweils das obere Ende 14 des Verdunstungskörpers 12 angebracht ist. Hierzu im Einzelnen:

Bei der in Fig. 7 im rechten Bildbereich gezeigten Ausführungsform der erfindungsgemäßen Raumbeduftungsvorrichtung 10 ist angrenzend bzw. seitlich vom Behälter 20 eine Halterung in Form eines Stativs 31 positioniert. Dieses Stativ 31 hat einen seitlichen Ausleger, dessen freies Ende bis oberhalb der Öffnung 21 des Behälters 20 reicht. Konkret wird der Verdunstungskörper 12 im Raum dadurch gehalten, dass sein oberes Ende 14 an dem seitlichen Ausleger des Stativs 31 angebracht bzw. befestigt ist. Hierbei ist die Positionierung des Stativs 31 relativ zum Behälter derart gewählt, dass das untere Ende 13 des Verdunstungskörpers 12 durch die Öffnung 21 hindurch in den Behälter 20 eingebracht ist und dadurch in die Flüssigkeit D, mit welcher der Behälter gefüllt ist (vgl. Fig. 5), eintaucht. In dieser Weise ist der Verdunstungskörper 13 der Raumbeduftungsvorrichtung 10 mit seiner Längserstreckung L₁₂ vertikal im Raum aufgehängt, wobei der Verdunstungskörper 13 in einem Längenabschnitt davon, der sich oberhalb der Öffnung 21 des Behälters 20 und somit außerhalb des Behälters 20 befindet, gegenüber der Umgebung exponiert ist und dort dann eine Verdunstung der zumindest einen Duftstoffe enthaltenen Flüssigkeit möglich ist.

Die in der Fig. 7 im linken Bildbereich gezeigte Ausführungsform der erfindungsgemäßen Raumbeduftungsvorrichtung 10 funktioniert im Prinzip genauso wie die zuletzt genannte Ausführungsform, mit dem Unterschied, dass hierbei anstatt eines separaten Stativs nunmehr an einer Wand des Raums R eine Halterung in Form eines Hakens 32 vorgesehen ist, an dem das obere Ende 14 des Verdunstungskörpers 12 angebracht ist. Die freie Länge dieses Hakens 32 und die Positionierung des Behälters 20 relativ zur Wand sind dahingehend abgestimmt, dass sich der Haken 32 mit seinem freien Ende in etwa oberhalb der Öffnung 21 des Behälters 20 befindet. Somit kann das obere Ende 14 des Verdunstungskörpers 12 an dem Haken 32 angebracht werden, wenn gleichzeitig das untere Ende 13 des Verdunstungskörpers 12 durch die Öffnung 21 in den Behälter 20 eingebracht ist und in die Flüssigkeit D eintaucht.

Alternativ zu einem Haken 32, der an der Wand eines Raums angebracht ist, kann für die zuletzt genannte Ausführungsform der erfindungsgemäßen Raumbeduftungsvorrichtung 10 auch eine geeignete Halterung an der Decke eines Raums vorgesehen sein. Hierbei ist dann das obere Ende 14 des Verdunstungskörpers 12 an dieser Halterung angebracht, um den Verdunstungskörper 12 hängend anzuordnen und dabei das untere Ende 13 in den Behälter 20 einzubringen.

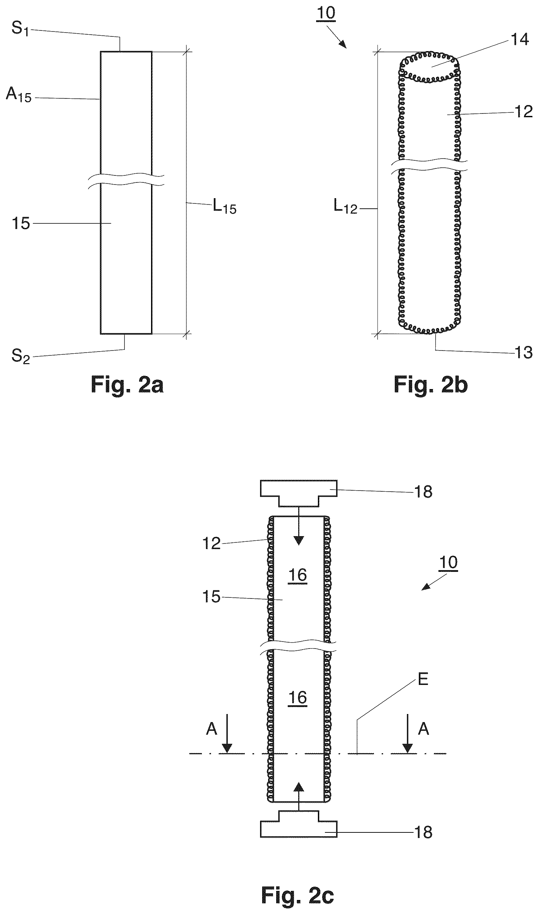

Nachstehend ist unter Bezugnahme auf die Fig. 2a-c, Fig. 3, Fig. 4 und Fig. 6 eine weitere Ausführungsform der erfindungsgemäßen Raumbeduftungsvorrichtung 10 gezeigt und erläutert, bei der ein stützendes längliches stabförmiges Element 15 verwendet wird. Hierzu im Einzelnen:
Fig. 2a zeigt das längliche stabförmige Element 15 vereinfacht in einer Seitenansicht. Die Längserstreckung dieses Elements 15 ist in der Fig. 2a mit "L₁₅" bezeichnet.

Das stabförmige Element 15 kann aus einem beliebigen Material hergestellt sein, beispielsweise aus Holz, Glas, Stein, Metall, Kunststoff, Keramik oder Gips, entweder, in einer Ebene senkrecht zur Längsachse gesehen, in einer massiven Ausführungsform, oder in Form eines Rohrs, das in einem Querschnitt senkrecht zur Längsachse hohl ausgebildet ist und entsprechend einen Innenraum 16 (vgl. Fig. 2c) aufweist. Jedenfalls ist für das stabförmige Element 15 erfindungsgemäß von Bedeutung, dass es dank der Auswahl eines geeigneten Materials hierfür eine ausreichende Stand- bzw. Knickfestigkeit aufweist, wenn es beispielsweise vertikal aufgestellt wird.

In Anpassung an das stabförmige Element 15, d.h. in etwa mit gleicher Länge und gleichem Durchmesser, ist bei dieser Ausführungsform der erfindungsgemäßen Raumbeduftungsvorrichtung 10 der zugehörige Verdunstungskörper 12 gewählt. Gemäß der Darstellung von Fig. 2b besteht ein solcher Verdunstungskörper 12 aus einem Textil-Material in Strumpfform. Somit ist ein solcher strumpfförmiger Verdunstungskörper 12 an bzw. über dem stabförmigen Element 15 an dessen Außenumfangsfläche A₁₅ (vgl. Fig. 2a) anbringbar.

Gemäß der Darstellung von Fig. 2c ist der Verdunstungskörper 12 von Fig. 2b in einem Zustand gezeigt, wenn er an der Außenumfangsfläche A₁₅ des stabförmigen Elements 15 angebracht ist.

Bei der Darstellung von Fig. 2c ist das stabförmige Element 15 in Form eines Rohrs ausgebildet, das entsprechend einen Innenraum 16 aufweist. Zweckmäßigerweise ist das stabförmige Element 15 in Form eines solchen Rohrs an seinen beiden Stirnseiten S₁ und S₂ jeweils offen ausgebildet.

Zwecks einer einfachen Befestigung des unteren Endes 13 und des oberen Endes 14 des Verdunstungskörpers 12 an dem stabförmigen Element 15 kann bei der Ausführungsform gemäß Fig. 2c vorgesehen sein, dass eine Längserstreckung L₁₂ des Verdunstungskörpers 12 größer als die Längserstreckung L₁₅ des stabförmigen Elements 15, mit der Folge, dass die endseitigen Abschnitte des Verdunstungskörpers 12, die an den Stirnseiten S₁ und S₂ (vgl. Fig. 2a) überstehen, dann einfach in diese offenen Stirnseiten S₁ und S₂ hineingestopft und dann unter Verwendung von jeweiligen Stopfen 18 an diesen Stirnseiten S₁ und S₂ des Rohrs 15 befestigt werden.

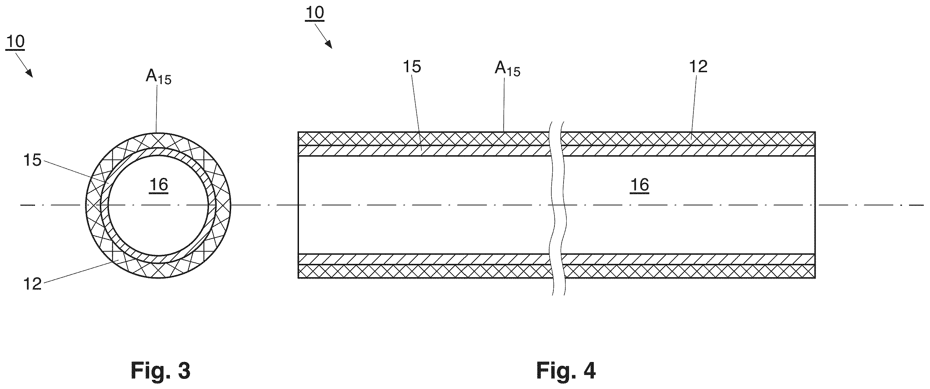

Die Ausgestaltung der zuletzt genannten Ausführungsform der erfindungsgemäßen Raumbeduftungsvorrichtung 10 in Bezug auf das hierbei eingesetzte stabförmige Element 15 in Form des Rohrs und des Verdunstungskörpers 12, der an der Außenumfangsfläche A₁₅ des Rohrs 15 angebracht ist, ist auch aus der Fig. 3 ersichtlich, die eine Querschnittansicht der Raumbeduftungsvorrichtung 10 entlang der Linie A-A von Fig. 2c zeigt. Hierbei ist u.a. zu erkennen, dass der Verdunstungskörper 12, nach Art eines Strumpfs, das Rohr 15 in einer Ebene senkrecht zu dessen Längsachse L₁₅ vollständig umschließt.

Fig. 4 zeigt die vorstehend genannte Ausführungsform der erfindungsgemäßen Raumbeduftungsvorrichtung 10 in einer Längsschnittansicht.

In Bezug auf die Schnittansichten gemäß Fig. 3 und Fig. 4 darf an dieser Stelle gesondert darauf hingewiesen werden, dass hierin das Material des Rohrs 15 schraffiert dargestellt ist, wobei das Textil-Material des Verdunstungskörpers 12, welches an der Außenumfangsfläche A₁₅ des Rohrs 15 angebracht ist, jeweils doppelt-schraffiert gezeigt ist.

Die vorstehend erläuterte Ausbildung des stabförmigen Elements 15 in Form eines Rohrs führt zu den Vorteilen einen relativ geringen Gewichts und von moderaten Herstellungskosten, und weiter vorteilhaft auch zu der Möglichkeit, dass - sofern die Stirnseiten eines solchen Rohrs offen ausgebildet sind - dann die endseitigen Abschnitte des Verdunstungskörpers 12 einfach in den offenen Stirnseiten S₁ und S₂ mittels geeigneter Stopfen 18 (vgl. Fig. 2c) befestigt werden können.

Die Darstellung gemäß Fig. 4 lässt ebenfalls erkennen, dass die Längserstreckung L₁₂ des des Verdunstungskörpers 12 an die Längserstreckung L₁₅ des Rohrs 15 derart gewählt ist, dass der Verdunstungskörper 12 die Außenumfangsfläche des Rohrs entlang von dessen Längserstreckung vollständig bedeckt. In dieser Weise ist für den praktischen Einsatz der erfindungsgemäßen Raumbeduftungsvorrichtung 10 eine möglichst große freie Oberfläche gewährleistet, an welcher die zumindest einen Duftstoff enthaltenen Flüssigkeit D bestimmungsgemäß verdunsten kann, um damit eine gewünschte Raumbeduftung zu erreichen.

Der praktische Einsatz der zuletzt genannten Ausführungsform der erfindungsgemäßen Raumbeduftungsvorrichtung 10 ist vereinfacht in der Fig. 6 gezeigt. Hierbei kann es sich um die Ausführungsform gemäß Fig. 2c handeln, gemäß der - wie erläutert - der Verdunstungskörper 12 nach Art eines Strumpfs an der Außenumfangsfläche A₁₅ des Rohrs 15 angebracht ist. Jedenfalls ist anhand der Fig. 6 erkennbar, dass der (an dem Rohr 15 angebrachte) Verdunstungskörper 12 mit seinem unteren Ende 13 (vgl. Fig. 2b) in die Öffnung 21 des Behälters 20 (vgl. Fig. 5) eingebracht ist, so dass das untere Ende 13 in die Flüssigkeit D eintaucht. In Folge dessen kann dann die Flüssigkeit D die Fasern F des Verdunstungskörpers 12 benetzen bzw. darin eindringen, was in der Fig. 6 durch zwei Pfeile angrenzend an das untere Ende 13 des Verdunstungskörpers 12 entsprechend symbolisiert ist. Im Anschluss hieran kommt es dank der Kapillarwirkung der Fasern des Verdunstungskörpers 12 zu einem Transport von Flüssigkeit D durch das Textil-Material hindurch in Richtung des oberen Endes 14 des Verdunstungskörpers 12, was in der Fig. 6 durch einen mit "K" bezeichneten Pfeil innerhalb des Verdunstungskörpers 12 kenntlich gemacht ist.

Schließlich wird in Bezug auf die zuletzt genannte Ausführungsform der erfindungsgemäßen Raumbeduftungsvorrichtung 10 darauf hingewiesen, dass das hierbei verwendete stabförmige Element 15, das - wie erläutert - in Form eines Rohrs ausgebildet sein kann, eine stützende Funktion für das Textil-Material und den hieraus gebildeten Verdunstungskörper 12 übernimmt. Dadurch, dass das Textil-Material an der Außenumfangsfläche A₁₅ des stabförmigen Element 15 bzw. Rohrs 15 angebracht ist, kann der Verdunstungskörper 12 die vorstehend bereits genannte Doppelfunktion erfüllen, nämlich sowohl den Transport der Flüssigkeit von dem ersten Ende 13 des Verdunstungskörpers 12, welches in dem Behälter 20 in die Flüssigkeit D eingetaucht ist, in Richtung des entgegengesetzten zweiten Endes 14 des Verdunstungskörpers 12, als auch das Verdunsten der Flüssigkeit D in einem Längenabschnitt davon, der sich oberhalb der Öffnung 21 des Behälters 20 und somit außerhalb des Behälters 20 befindet, gegenüber der Umgebung exponiert ist. In der Darstellung von Fig. 6 ist dieser exponierte Längenabschnitt des Verdunstungskörpers 12 mit "L_{E}" bezeichnet.

### Bezugszeichenliste

- 10: Raumbeduftungsvorrichtung
- 12: Verdunstungskörper
- 13: unteres Ende (des Verdunstungskörpers 12)
- 14: oberes Ende (des Verdunstungskörpers 12)
- 15: längliches stabförmiges Element (z.B. in Form eines Rohrs)
- 16: Innenraum (des stabförmigen Elements in Form eines Rohrs 15)
- 18: Stopfen
- 20: Behälter
- 21: Öffnung (des Behälters 20)
- 31: Stativ
- 32: Haken (z.B. an einer Raumdecke oder an einer Wand)
- A₁₅: Außenumfangsfläche (des stabförmigen Elements 15)
- D: Duftstoff enthaltende Flüssigkeit
- E: Ebene (senkrecht zur Längsachse des stabförmigen Elements 15)
- F: Fasern (des Verdunstungskörpers 12)
- K: Kapillarwirkung (innerhalb des Verdunstungskörpers 12)
- L_{E}: exponierter Längenabschnitt (des Verdunstungskörpers 12)
- L₁₂: Längserstreckung (des Verdunstungskörpers 12)
- L₁₅: Längsachse (des stabförmigen Elements 15 bzw. des Rohrs)
- R: Raum (innerhalb eines Gebäudes)
- S₁: obere Stirnseite (des stabförmigen Elements 15)
- S₂: untere Stirnseite (des stabförmigen Elements 15)

## Patentansprüche

1. Raumbeduftungsvorrichtung (10) zur Abgabe eines Duftstoffs an die Umgebung insbesondere in Form von geschlossenen Räumen (R) in Gebäuden, umfassend einen Verdunstungskörper (12) mit einer Vielzahl von Fasern (F), wobei der Verdunstungskörper (12) eine Kapillarwirkung (K) aufweist und zur Aufnahme von Duftstoffen in flüssiger Form geeignet ist,
**dadurch gekennzeichnet,**
**dass** der Verdunstungskörper (12) aus einem Textil-Material gebildet ist oder zumindest ein Textil-Material aufweist.

2. Raumbeduftungsvorrichtung (10) nach Anspruch 1, **dadurch gekennzeichnet, dass** der Verdunstungskörper (12) eine Längserstreckung (L₁₂) aufweist, wobei die Kapillarwirkung (K) des Verdunstungskörpers (12) zumindest entlang seiner Längserstreckung (L₁₂) vorliegt.

3. Raumbeduftungsvorrichtung (10) nach Anspruch 1 oder 2, **gekennzeichnet durch** ein längliches stabförmiges Element (15), wobei der Verdunstungskörper (12) in Form des Textil-Materials an einer Außenumfangsfläche (A₁₅) des stabförmigen Elements (15) angebracht ist, so dass der Verdunstungskörper (12) gegenüber der Umgebung exponiert ist, vorzugsweise, dass der Verdunstungskörper (12) in Form des Textil-Materials die Außenumfangsfläche (A₁₅) des stabförmigen Elements (15) entlang von dessen Längsachse (L₁₅) vollständig bedeckt.

4. Raumbeduftungsvorrichtung (10) nach Anspruch 3, **dadurch gekennzeichnet, dass** der Verdunstungskörper (12) in Form des Textil-Materials das stabförmige Element (15) in einer Ebene (E) senkrecht zu dessen Längsachse (L₁₅) vollständig umschließt.

5. Raumbeduftungsvorrichtung (10) nach Anspruch 3 oder 4, **dadurch gekennzeichnet, dass** das stabförmige Element (15) zumindest in einem Segment entlang seiner Längsachse (L₁₅) ein im Querschnitt hohl ausgebildetes Rohr ist, vorzugsweise, dass das stabförmige Element (15) entlang seiner gesamten Längsachse (L₁₅) ein im Querschnitt hohl ausgebildetes Rohr ist.

6. Raumbeduftungsvorrichtung (10) nach Anspruch 5, **dadurch gekennzeichnet, dass** der Verdunstungskörper (12) in Form des Textil-Materials an zumindest einer Stirnseite (S₁, S₂) des stabförmigen Elements (15) in Form eines Rohrs in einen Innenraum (16) des Rohrs (15) gedrückt und an der Stirnseite (S₁, S₂) des Rohrs (15) mittels eines Stopfens (18) befestigt ist.

7. Raumbeduftungsvorrichtung (10) nach Anspruch 2, **dadurch gekennzeichnet, dass** die Fasern (F) des Verdunstungskörpers (12) in Form des Textil-Materials derart beschaffen sind, dass der Verdunstungskörper (12), falls er mit seiner Längserstreckung (L₁₂) vertikal aufgestellt ist, in Richtung seiner Längserstreckung (L₁₂) eine ausreichende Knickfestigkeit aufweist und dadurch selbstständig standfest ist.

8. Raumbeduftungsvorrichtung (10) nach einem der vorhergehenden Ansprüche, **dadurch gekennzeichnet, dass** das Textil-Material Naturfasern, Kunstfasern und/oder Glasfasern umfasst.

9. Raumbeduftungsvorrichtung (10) nach einem der vorhergehenden Ansprüche, **gekennzeichnet durch** einen Behälter (20) für eine zumindest einen Duftstoff enthaltende Flüssigkeit, wobei der Verdunstungskörper (12) in Form des Textil-Materials mit seinem unteren Ende (13) in eine Öffnung (21) des Behälters (20) einbringbar ist und mit seinem oberen Ende (14) aus der Öffnung (21) des Behälters (20) herausragt.

10. Raumbeduftungsvorrichtung (10) nach Anspruch 9, **gekennzeichnet durch** eine Halterung (31, 32), an der ein oberes Ende (13) des Verdunstungskörpers (12) in Form des Textil-Materials, welches aus der Öffnung (21) des Behälters (20) herausragt, angebracht ist, vorzugsweise, dass die Halterung in Form eines Stativs (31), welches angrenzend zum Behälter (20) positionierbar ist und eine größere Höhe als der Behälter (20) aufweist, oder in Form eines an einer Raumdecke oder an einer Wand angebrachten Hakens (32) ausgebildet ist.

11. Verfahren zur Raumbeduftung von Räumen (R) in Gebäuden, bei dem ein Verdunstungskörper (12), der aus einem Textil-Material gebildet ist oder zumindest ein Textil-Material aufweist und dank einer Vielzahl von Fasern (F) entlang seiner Längserstreckung (L₁₂) eine Kapillarwirkung (K) aufweist, mit seinem unteren Ende (13) in eine Öffnung (21) eines Behälters (20), der mit einer zumindest einen Duftstoff enthaltenden Flüssigkeit gefüllt ist, eingebracht wird und mit seinem entgegengesetzten oberen Ende (14) aus der Öffnung (21) des Behälters (20) herausragt, wobei der Verdunstungskörper (12) in einem Längenabschnitt (L_{E}) davon, der sich oberhalb der Öffnung (21) des Behälters (20) und somit außerhalb des Behälters (20) befindet, gegenüber der Umgebung exponiert ist.

12. Verfahren nach Anspruch 11, **dadurch gekennzeichnet, dass** dieses Verfahren mit einer Raumbeduftungsvorrichtung (10) nach einem der Ansprüche 1 bis 10 durchgeführt wird.
